# NOUVEAU FASCICULE DE BREVET EUROPEEN

(11) **EP 0 723 776 B2**
(45) Date de publication et mention de la décision concernant l'opposition: **29.04.2009**
(45) Mention de la délivrance du brevet: 15.01.1997
(21) Numéro de dépôt: 96400057.4
(22) Date de dépôt: 10.01.1996
(51) Int. Cl.: A61Q 1/12, A61K 8/37, A61K 8/891, A61K 8/897, A61K 8/898, A61Q 1/06, A61Q 1/10

(54) **Composition cosmétique comprenant un composé siliconé et un ester d'acide gras**
Kosmetische Zusammensetzung, die eine Silikonverbindung und einen Fettsäureester enthält
Cosmetic composition containing a silicone compound and a fatty acid ester

(30) Priorité: 30.01.1995 FR 9501040
(43) Date de publication de la demande: 31.07.1996
(73) Titulaire: L'ORÉAL, 75008 Paris (FR)
(72) Inventeur: Mellul, Myriam, F-94240 L'Hay les Roses (FR); Arnaud, Pascal, F-94000 Creteil (FR)
(74) Mandataire: Bulle, Françoise

(56) Documents cités:
- EP-A2- 0 437 216
- EP-A2- 0 602 905
- WO-A1-94//18940
- US-A- 5 085 855
- US-A- 5 116 604
- 'CTFA Cosmetic Ingredient Dictionary', 1982
- 'Handbook of Cosmetic and Personal Care Additives,', 1994, GOWER, ISBN 0566074702 article MICHAEL AND IRENE ASH
- 'The Condensed Chemichal Dictionary "hydrocarbon"', vol. 9, VAN NOSTRAND REINHOLD, ISBN 044223240-3 pages 449 - 449
- 'CFC Handbook of Phisic & Chemistry "hydrocarbon"', 1957 pages 1 - 32
- 'CTFA Cosmetic Ingredient Handbook', vol. 2, 1992, THE COSMETIC, TOILETR AND FRAGANCE ASSOCIATION, ISBN 1882621DOX pages 608-609 - 621-641
- 'Introduction to Organic and Biological Chemistry', 1970, THE MACMILLAN COMPANY pages 248 - 248
- 'Décodage et fiche produit MYME: POLYMIST B-12 Allied Chemical' 1980 - 1982,
- 'Spécification Allied Signal ACUMIST (11/93)' Novembre 1993,
- 'Brochure Allied Signal (avril 1994)-ACUMIST' Avril 1994,
- 'Information Dow Corning Silicones Volatiles Fluides (1982)' 1982,
- 'Stick Product, Soap/Cosmetics/Chemical Specialtis' Juin 1987,
- 'Lipstick:History as a Window to the Future', vol. 101, Avril 1986, COSMAIS, INC.CLARK, NEW JERSEY article GEOFF FINKENAUR, pages 50 - 55
- 'Maquillage des Lèvres ,Encycl Méd Chir, Cosmétolgie, 50-170-C-10', 2001 article H.PAWIN ET M.VERSHOORE, pages 50 - 170
- 'Maquillage du teint du visage,Encycl Méd Chir, Cosmétologie,50-170-A-10', 2001
- 'Harry's Cosmeticology' 1973, pages 165 - 170
- EXXON CHEMICALS: 'Les acides en un coup d'oeil,'
- 'Dow Corning 200 Fluids'
- 'Wacker-Belsil PDM 20,PDM 200,PDM 1000 Phenyl Trimethicome'
- 'Tableau récapitulatif des résines silicones, Dow Corning 593'

## Description

La présente invention a trait à une composition cosmétique comprenant des composés siliconés tels que des huiles, gommes et/ou cires de silicone, et des composés hydrocarbonés, susceptible d'être utilisée comme composition de maquillage et/ou de soin de la peau.

L'utilisation de composés siliconés dans des compositions cosmétiques, notamment de maquillage, est connue. Ces composés permettent, entre autre, d'obtenir sur la peau un film particulièrement homogène, possédant de bonnes propriétés cosmétiques. Ils permettent également d'améliorer la tenue du maquillage de part leur caractère hydrophobe.
Il est également connu d'utiliser des composés hydrocarbonés dans les compositions cosmétiques.
Toutefois, on a constaté que certains composés siliconés étaient incompatibles avec certains composés hydrocarbonés généralement utilisés dans les compositions cosmétiques, ce qui en limitait l'utilisation. Diverses solutions ont été proposées afin de permettre la préparation d'une composition cosmétique comprenant à la fois des composés siliconés et des composés hydrocarbonés. On peut citer, par exemple, l'utilisation de solvants ou co-solvants hydrocarbonés tels que les isoparaffines; toutefois, leur odeur et leur volatilité ne sont pas toujours appréciées.
On peut également citer la demande de brevet JP 62-169714 qui décrit une composition comprenant une huile de silicone et un corps gras solide, dans laquelle il est nécessaire, d'une part, d'ajouter lors de sa préparation, une huile d'ester à point de solidification égal ou supérieur à 0°C, possédant une structure intramoléculaire ramifiée constituée d'un acide gras et d'un alcool, et, d'autre part, de traiter la charge pigmentaire avec un méthylhydrogénopolysiloxane. On peut encore citer la demande EP437216 qui décrit des agents solubilisants et/ou dissolvants des silicones se présentant sous forme d'esters de formule R₁COOR₂ où R₁ est un isoalkyle en C₄₋₁₇ et R₂ est un isoalkyle en C₃₋₁₈.
Toutefois, les compositions cosmétiques comprenant ces agents solubilisants apportent, lorsqu'on les applique sur la peau, une sensation de sécheresse particulièrement désagréable, en particulier dans le cas des rouges à lèvres.

Le but de la présente invention est de proposer une composition cosmétique homogène, comprenant au moins un composé siliconé en association avec un composé hydrocarboné de masse moléculaire élevée.

La présente invention a donc pour objet une composition cosmétique anhydre comprenant dans une phase grasse, au moins un composé siliconé en association avec de l'octyldodécyl néopentanoate et un composé hydrocarboné autre que l'octyldodécyl néopentanoate choisi parmi les cires végétales, animales, minérales et/ou synthétiques.
On a en effet constaté que, de manière surprenante et inattendue, une telle composition se présente sous forme homogène, c'est-à-dire que, dans le cas de mélanges liquide/liquide, on observe une distribution homogène des constituants, d'où un mélange à une seule phase, et que, dans le cas de mélanges solide/liquide, tels que cire-dans-huile, on observe la formation d'un mélange homogène et limpide à chaud et la formation d'une dispersion homogène de la cire dans l'huile lors du refroidissement.
La composition obtenue présente comme avantage de posséder de bonnes propriétés cosmétiques, et en particulier ne procure pas de sensation de sécheresse lorsqu'elle est appliquée sur la peau.
Un autre avantage de l'invention réside dans le fait que l'on peut ajouter dans la phase grasse d'autres composés siliconés et/ou hydrocarbonés, généralement incompatibles entre eux, tout en gardant une composition homogène.

Un autre objet de l'invention est l'utilisation de l'octyldodécyl néopentanoate en tant qu'agent compatibilisant dans une composition cosmétique anhydre comprenant au moins un composé siliconé et un composé hydrocarboné autre que l'octyldodécyl néopentanoate choisi parmi les cires végétales, animales, minérales et/ou synthétiques.
Par agent compatibilisant, on entend dans la présente description, un agent qui permet d'obtenir une composition homogène telle que définie ci-dessus.

L'octyldodécyl néopentanoate est un ester d'acide néopentanoïque en C₅, et d'alcool isoarachidylique en C₂₀. On a donc constaté que cet ester était un excellent agent compatibilisant des composés siliconés entre eux, et/ou des composés siliconés avec des composés hydrocarbonés. De plus, l'octyldodécyl néopentanoate présente une bonne stabilité thermique et chimique, et permet l'obtention de compositions de texture beaucoup plus huileuse à l'application, en comparaison avec des compositions de l'art antérieur ne le comprenant pas.
Enfin, on a constaté que l'octyldodécyl néopentanoate présente des propriétés dispersantes vis-à-vis des poudres remarquables et permet l'obtention d'une dispersion homogène.

L'octyldodécyl néopentanoate peut représenter 0,5-99% en poids, de préférence 1-70% en poids, de la phase grasse de la composition selon l'invention.

D'une manière générale, la composition selon l'invention peut se présenter sous forme d'un produit compacté ou coulé, ou encore sous forme d'un produit gras anhydre, solide ou liquide.
Elle comprend donc une phase grasse dans laquelle sont présents des composés siliconés et hydrocarbonés, l'octyldodécyl néopentanoate et des éventuels additifs lipophiles voire hydrophiles, ainsi qu'éventuellement une phase pulvérulente comprenant des pigments et/ou des charges.
La phase grasse peut représenter 1-100% en poids de la composition finale et la phase pulvérulente peut représenter 0-99% en poids de la composition finale.

La composition selon l'invention comprend donc au moins un composé siliconé qui peut être choisi parmi les huiles, les gommes et/ou les cires de silicone.
On peut en particulier citer les cyclométhicones telles que les cyclométhicones D4, D5, D6; les polydiméthylsiloxanes; les alkyldiméthicones; les polyphénylméthylsiloxanes tels que les phényldiméthicones et les phényltriméthicones; ainsi que les silicones modifiés par des groupements aliphatiques et/ou aromatiques, éventuellement fluorés, ou par des groupements fonctionnels tels que des groupements hydroxyles, thiols et/ou amines. On peut également citer les silicones de formule (I): 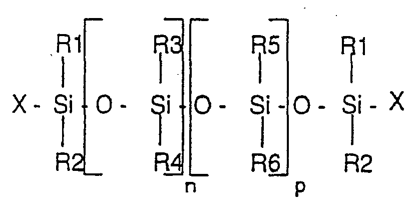 dans laquelle :
R₁, R₂, R₅ et R₆ sont, ensemble ou séparément, un radical alkyle ayant 1 à 6 atomes de carbone,
R₃ et R₄ sont, ensemble ou séparément, un radical alkyle ayant de 1 à 6 atomes de carbone, ou un radical aryle, X est un radical alkyle ayant de 1 à 6 atomes de carbone, un radical hydroxyle ou un radical vinyle,
n et p étant choisis de manière à conférer au composé siliconé une viscosité supérieure à 100 000 mPa.s, de préférence supérieure à 500 000 mPa.s et pouvant aller jusqu'à 1 000 000 mPa.s.

La composition selon l'invention peut également comprendre des composés hydrocarbonés tels que les huiles végétales, animales, minérales et/ou synthétiques.
On peut citer l'huile de paraffine, de vaseline, le perhydrosqualène, l'huile d'arara, l'huile d'amande douce, de calophyllum, d'avocat, de sésame, de ricin, de jojoba, d'olive ou de germes de céréales. On peut également utiliser des esters d'acide lanolique, d'acide oléique, d'acide laurique, d'acide stéarique ou d'acide myristique par exemple; des alcools tels que l'alcool oléique, l'alcool linoléique ou linolénique, l'alcool isostéarique ou l'octyl dodécanol; des acétylglycérides, des octanoates, décanoates ou ricinoléates d'alcools ou de polyalcools, les triglycérides caprylique/caprique, les triglycérides d'acides gras en C₁₀ à à C₁₈.
On peut encore utiliser des huiles hydrogénées concrètes à 25°C telles que les huiles de ricin, de palme ou de coco hydrogénées, ou le suif hydrogéné; des mono-, di-, tri- ou sucro- glycérides; des lanolines; des esters gras concrets à 25°C. Parmi les cires hydrocarbonées, on peut citer les cires animales telles que la cire d'abeilles; les cires végétales telles que la cire de Carnauba, de Candellila, d'Ouricoury, du Japon ou les cires de fibres de lièges ou de canne à sucre; les cires minérales, par exemple de paraffine, de lignite ou les cires microcristallines ou les ozokérites; les cires synthétiques, parmi lesquelles les cires de polyéthylène et les cires obtenues par synthèse de Fscher-Tropsch.

La composition peut également comprendre des pigments et/ou des charges habituellement utilisées dans de telles compositions cosmétiques.
Les pigments peuvent être blancs ou colorés, minéraux, organiques et/ou nacrés. On peut citer, sans effets limitatifs, le dioxyde de titane, l'oxyde de zinc, le dioxyde de zirconium, les oxydes de fer noir, jaune, rouge, brun, le dioxyde de cérium, l'oxyde de chrome, le bleu ferrique, le noir de carbone, les laques de baryum, strontium, calcium, aluminium ainsi que le mica recouvert d'oxyde de titane ou d'oxychlorure de bismuth.
Les charges peuvent être minérales, organiques ou de synthèse, lamellaires ou non lamellaires, sphériques ou non sphériques. On peut citer le talc, le mica, la silice, le kaolin, les poudres de Nylon et de polyéthylène, le Téflon, l'amidon, le micatitane, la nacre naturelle, le nitrure de bore, les microsphères creuses telles que l'Expancel de Nobel Industrie, et les microbilles de résine de silicone.

La composition selon l'invention peut également comprendre des constituants habituellement utilisés dans ce genre de compositions cosmétiques.

Ces constituants sont de préférence choisis en fonction de l'effet cosmétique souhaité pour la composition finale, tel que la couvrance, la transparence, la matité et/ou l'aspect satiné. On peut citer, sans effets limitatifs :
· les gélifiants comme les argiles modifiées connues sous les noms de bentone, vendues par la société NL Industrie et utilisées telles qu'elles ou préalablement conditionnées dans un gel; la silice hydrophobe; les sels gras d'aluminium.
· les vitamines comme les tocophérols et leurs dérivés, la vitamine A et ses dérivés, la vitamines C et ses dérivés comme les esters gras dont le palmitate.
· les filtres solaires comme l'octylméthoxycinnamate (Parsol MCX), la 3-benzophénone (Uvinul M40), le butylméthoxydibenzoyl-méthane (Parsol 1789).
· les matériaux huileux comme les parfums, les huiles essentielles, les huiles fluorées.
· les agents hydratants tels que le propylène glycol et le glycérol.

Les constituants hydrophiles sont de préférence dispersés dans la phase grasse.

Les procédés de fabrication des compositions selon l'invention ne différent en rien des procédés classiquement utilisés en cosmétique et parfaitement connus de l'homme de l'art.

Les compositions selon l'invention peuvent se présenter sous forme d'un produit de soin et/ou de maquillage de la peau et/ou des matières kératiniques.
Ce produit peut être sous forme d'une poudre libre, coulée ou compactée (fond de teint, fard à joues, fard à paupières), d'un produit gras anhydre plus ou moins fluide (rouge à lèvres, mascara, vernis solvant), d'une huile ou lotion pour le corps et/ou le visage, voire d'un produit capillaire tel qu'un gel anhydre coiffant.

L'invention est illustrée plus en détail dans les exemples suivants, dans lesquels les pourcentages sont donnés en poids.

### Exemple 1

On compare la solubilité d'une huile et d'une gomme de silicone dans l'ester selon l'invention et dans des esters de l'art antérieur de structure chimique proche. L'huile de silicone est un PDMS de viscosité 350 mPa.s (huile DC200 de Dow Corning)
La gomme de silicone est une gomme de formule (I) dans laquelle les substituants R1 à R6 et X représentent un groupement méthyle, p = 0 et n = 2300, de viscosité 500 000 mPa.s (AK 500 000 de Waker).

On obtient les résultats suivants :

| | Nombre d'atomes de carbone | huile de silicone | gomme de silicone |
|---|---|---|---|
| Octyldodécyl néopentanoate (Elefac I-205 de Bernel Chemical) | 20 + 5 | ∞ | ∞ |
| Isostéaryl néopentanoate (Ceraphyl 375 de ISP) | 18 + 5 | 0 | 0 |
| Hexyl-2 décyl isononanoate (Stéarineries Dubois) | 16 + 9 | 0 | 0 |
| Isostéaryl isononanoate (Lanol 189 de SEPPIC) | 18 + 9 | 0 | 0 |

| | | | |
|---|---|---|---|
| ∞: on constate une excellente solubilité de l'huile et de la gomme dans l'ester considéré, en toute proportion 0: on ne constate aucune solubilité de l'huile ou de la gomme dans l'ester considéré | | | |

L'octyldodécyl néopentanoate permet d'obtenir une composition homogène comprenant l'ester considéré et une huile ou une gomme de silicone particulières, alors que des esters de structure chimique proche ne le permettent pas.

### Exemple 2

On mesure le faux massique maximum d'ester que l'on peut ajouter, à 25°C, dans une solution de gomme dans une huile de silicone, sans observer de précipitation de la gomme.
La solution testée comprend une gomme de formule (I) dans laquelle les substituants R1 à R6 représentent un groupement méthyle, le substituant X représente un groupement hydroxyle, p = 0 et n = 2700, en solution à 13% dans du polydiméthylsiloxane (Q2-1403 de Dow Corning).

On obtient les résultats suivants :

| Octyldodécyl néopentanoate (Elefac I-205 de Bernel Chemical) | pas de précipitation au moins jusqu'à 1000% |
|---|---|
| Isostéaryl néopentanoate (Ceraphyl 375 de ISP) | 68% |
| Hexyl-2 décyl isononanoate (Stéarineries Dubois) | 72% |
| Isostéaryl isononanoate (Lanol 189 de SEPPIC) | 60% |

On constate que l'octyldodécyl néopentanoate permet de solubiliser quasiment à l'infini une gomme particulière, alors que des esters de structure chimique proche ne le permettent pas.

### Exemple 3

On observe la bonne compatibilité de l'octyldodécyl néopentanoate avec plusieurs composés siliconés de structure chimique différentes.

On obtient les résultats suivants :

| | |
|---|---|
| Huile de silicone (PDMS) DC200 de Dow Corning 350 mPa.s | ∞ |
| Phényl triméthicone BELSIL PDM 1000 de Wacker | ∞ |
| Diphényldiméthicone Gomme 761 de Rhône Poulenc | supérieur à 10% |
| Alkyl diméthicone D2 5519 de Dow Corning | ∞ |
| Fluorodiméthicone GRANSIL DM 100 de Grant | ∞ |
| Amodiméthicone L656 de Wacker | ∞ |
| Silicone hydroxylée SILBIONE 71516V60 de Rhône-Poulenc | ∞ |
| Mélange PDMS + triméthylsiloxysilicate (67/33) DC593 de Dow Corning | ∞ |

L'octyldodécyl néopentanoate présente donc une compatibilité certaine avec un grand nombre de composés siliconés de structures chimiques diverses.

### Exemple 4

On prépare un mélange comprenant :
- 30% de mélange PDMS + triméthylsiloxysilicate (DC593 de Dow Corning)
- 30% d'alkyldiméthicone (ABIL WAX 9801 de Goldschmidt) et
- 40% d'octyldodécyl néopentanoate

On obtient une solution homogène, alors qu'en l'absence d'octyldodécyl néopentanoate, on observe, de manière macroscopique et microscopique, un mélange non homogène des constituants. L'octyldodécyl néopentanoate peut donc être utilisé comme co-solvant de silicones non compatibles entre elles.

### Exemple 5

On prépare un rouge à lèvres comprenant:

| | |
|---|---|
| . octyldodécyl néopentanoate | 60% |
| . diphényldiméthicone (Gomme 761 de Rhône Poulenc) | 0,1% |
| . cire de polyéthylène | 13,5% |
| . cire microcristalline | 4,5% |
| . propionate d'arachidyle | 10% |
| . pigments | 11,9% |

On dissout la gomme de silicone à 80°C dans l'octyldodécyl néopentanoate. Après homogénéisation, on ajoute, à 95°C, les autres constituants.
Après agitation et broyage, le mélange est coulé à 95°C dans des alvéoles, de manière à obtenir un rouge à lèvres présentant de bonnes propriétés cosmétiques. En particulier, l'on n'observe aucune sensation de sec sur les lèvres lors de l'application du rouge, ce qui est un aspect sensoriellement intéressant de la composition selon l'invention.

### Exemple 6

On prépare un rouge à lèvres comprenant :

| | |
|---|---|
| . octyldodécyl néopentanoate | 65% |
| . alkyldiméthicone (D2 5519 de Dow Corning) | 5% |
| . cire de polyéthylène | 13,5% |
| . cire microcristalline | 4,5% |
| . pigments | 12% |

L'ensemble des constituants est mélangé à 95°C.

Après homogénéisation et broyage, le mélange est coulé à 95°C dans des alvéoles, de manière à obtenir un rouge à lèvres présentant de bonnes propriétés cosmétiques.

## Revendications

1. Composition cosmétique anhydre comprenant dans une phase grasse au moins un composé siliconé de l'octyldodécyl néopentanoate et un composé hydrocarboné autre que l'octyldodécyl néopentanoate choisi parmi les cires végétales, animales, minérales et/ou synthétiques.

2. Composition selon la revendication 1, dans laquelle la phase grasse comprend en outre au moins un second composé siliconé

3. Composition selon l'une des revendications précédentes, dans laquelle l'octyldodécyl néopentanoate représente 0,5-99% en poids, de préférence 1-70% en poids, du poids de la phase grasse.

4. Composition selon l'une des revendications précédentes, comprenant 1-100% en poids de phase grasse comprenant l'octyldodécyl néopentanoate et les composés siliconés et hydrocarbonés et 0-99% en poids d'une phase pulvérulente.

5. Composition selon l'une des revendications précédentes, dans laquelle les composés siliconés sont choisis parmi les cyclométhicones telles que les cyclométhicones D4, D5, D6; les polydiméthylsiloxanes; les alkyldiméthicones; les polyphénylméthylsiloxanes tels que les phényldiméthicones et les phényltriméthicones; les silicones modifiés par des groupements aliphatiques et/ou aromatiques, éventuellement fluorés, ou par des groupements fonctionnels tels que des groupements hydroxyles, thiols et/ou amines; les silicones de formule (I): 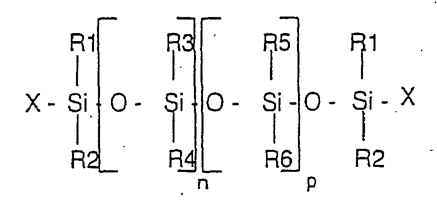 dans laquelle :
R₁, R₂, R₅ et R₆ sont, ensemble ou séparément, un radical alkyle ayant 1 à 6 atomes de carbone,
R₃ et R₄ sont, ensemble ou séparément, un radical alkyle ayant de 1 à 6 atomes de carbone, ou un radical aryle,
X est un radical alkyle ayant de 1 à 6 atomes de carbone, un radical hydroxyle ou un radical vinyle,
n et p étant choisis de manière à conférer au composé siliconé une viscosité supérieure à 100 000 mPa.s.

6. Composition selon l'une des revendications 1 à 5, dans laquelle le composé hydrocarboné est choisi parmi la cire d'abeilles, la cire de Carnauba, de Candellila, d'Ouricoury, du Japon ou les cires de fibres de lièges ou de canne à sucre; les cires de paraffine, de lignite ou les cires microcristallines ou les ozokérites; les cires de polyéthylène et les cires obtenues par synthèse de Fischer-Tropsch.

7. Composition selon l'une des revendications précédentes, dans laquelle la composition comprend des pigments et/ou des charges.

8. Composition selon la revendication 7, dans laquelle les pigments sont choisis parmi les pigments minéraux, organiques et/ou nacrés.

9. Compostion selon la revendication 7, dans laquelle les charges sont choisies parmi les charges minérales, organiques ou de synthèse, lamellaires ou non lamellaires, sphériques ou non sphériques.

10. Composition selon l'une des revendications 7 à 9, dans laquelle les pigments et/ou les charges sont choisies parmi le dioxyde de titane, l'oxyde de zinc, le dioxyde de zirconium, les oxydes de fer, le dioxyde de cérium, l'oxyde de chrome, le bleu ferrique; le noir de carbone, les laques de baryum, strontium, calcium, aluminium; le mica recouvert d'oxyde de titane ou d'oxychlorure de bismuth; le talc, le mica, la silice, le kaolin, les poudres de Nylon et de polyéthylène, le Téflon, l'amidon, le micatitane, la nacre naturelle, le nitrure de bore, les microsphères creuses et les microbilles de résine de silicone.

11. Composition selon l'une des revendications précédentes, se présentant sous forme d'un produit de soin et/ou de maquillage de la peau et/ou des matières kératiniques, tel qu'une poudre libre, coulée ou compactée (fond de teint, fard à joues, fard à paupières), un produit gras anhydre plus ou moins fluide (rouge à lèvres, mascara, vernis solvant), une huile ou lotion pour le corps et/ou le visage, et un produit capillaire comme un gel anhydre coiffant.

12. Utilisation de l'octyldodécyl néopentanoate en tant qu'agent compatibilisant dans une composition cosmétique anhydre comprenant au moins un composé siliconé et un composé hydrocarboné autre que l'octyldodécyl néopentanoate choisi parmi les cires végétales, animales, minérales et/ou synthétiques.

## Claims

1. Anhydrous cosmetic composition comprising, in a fatty phase, at least one silicone-containing compound, octyl-dodecyl neopentanoate and one hydrocarbon compound other than octyldodecyl neopentanoate chosen from plant, animal, mineral and/or synthetic oils or waxes.

2. Composition according to claim 1, in which the fatty phase also comprises at least one second silicone-containing compound.

3. Composition according to either of the preceding claims, in which the octyldodecyl neopentanoate represents 0.5-99% by weight, preferably 1-70% by weight, of the weight of the fatty phase.

4. Composition according to one of the preceding claims, comprising 1-100% by weight of fatty phase comprising the octyldodecyl neopentanoate and the silicone-containing compounds and hydrocarbon compounds and 0-99% by weight of a pulverulent phase.

5. Composition according to one of the preceding claims, in which the silicone-containing compounds are chosen from cyclomethicones such as cyclomethicones D4, D5 and D6; polydimethylsiloxanes; alkyldimethicones; polyphenylmethylsiloxanes such as phenyldimethicones and phenyltrimethicones; and silicones modified with aliphatic and/or aromatic groups, which optionally contain fluorine, or with functional groups such as hydroxyl, thiol and/or amine groups; the silicones of formula (I): 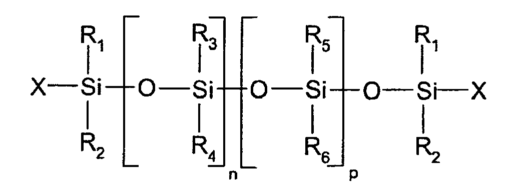 in which:
R₁, R₂, R₅ and R₆ are, together or separately, an alkyl radical having 1 to 6 carbon atoms,
R₃ and R₄ are, together or separately, an alkyl radical having from I to 6 carbon atoms or an aryl radical,
X is an alkyl radical having from 1 to 6 carbon atoms, a hydroxyl radical or a vinyl radical,
n and p being chosen so as to impart to the silicone-containing compound a viscosity of greater than 100,000 mPas.

6. Composition according to one of claims 1 to 5, in which the hydrocarbon compound is chosen from beeswax, carnauba wax, candellila wax, ouricurry wax, Japan wax or waxes from cork fibres or from sugar cane; paraffin wax, lignite wax or microcrystalline waxes or ozokerites; polyethylene waxes and waxes obtained by Fischer-Tropsch synthesis.

7. Composition according to one of the preceding claims, in which the composition comprises pigments and/or fillers.

8. Composition according to Claim 7, in which the pigments are chosen from mineral, organic and/or pearlescent pigments.

9. Composition according to Claim 7, in which the fillers are chosen from inorganic, organic or synthetic, lamellar or non-lamellar and spherical or non-spherical fillers.

10. Composition according to either of Claims 7 to 9, in which the pigments and/or fillers are chosen from titanium dioxide, zinc oxide, zirconium dioxide, iron oxides, cerium dioxide, chromium oxide, ferric blue; carbon black, barium, strontium, calcium and aluminum lakes; and mica coated with titanium oxide or with bismuth oxychloride; talc, mica, silica, kaolin, Nylon and polyethylene powders, Teflon, starch, titanium mica, natural mother of pearl, boron nitride, hollow microspheres and silicone resin microbeads.

11. Composition according to one of the preceding claims, in the form of a care product and/or a make-up product for the skin and/or keratinous materials such as a free, poured or compacted powder (foundation, blusher or eye-shadow), a more or less fluid anhydrous greasy product (lipstick, mascara or solvent varnish), an oil or lotion for the body and/or the face, or a hair product such as an anhydrous styling gel.

12. Use of octyldodecyl neopentanoate as a compatibilizing agent in an anhydrous cosmetic composition comprising at least one silicone-containing compound and one hydrocarbon compound other than octyldodecyl neopentanoate chosen from plant, animal, mineral and/or synthetic oils or waxes.

## Patentansprüche

1. Wasserfreie kosmetische Zusammensetzung, die in einer Fettphase mindestens eine Siliconverbindung, Octyldodecylneopentanoat und eine Kohlenwasserstoffverbindung enthält, die von Octyldodecylneopentanoat verschieden ist und ausgewählt ist unter pflanzlichen, tierischen, mineralischen und/oder synthetischen Ölen oder Wachsen.

2. Zusammensetzung nach Anspruch 1, die in der Fettphase ferner mindestens eine zweite Siliconverbindung enthält.

3. Zusammensetzung nach einem der vorhergehenden Ansprüche, in der das Octyldodecylneopentanoat 0,5 bis 99 Gew.-% und vorzugsweise 1 bis 70 Gew.-% des Gewichts der Fettphase ausmacht.

4. Zusammensetzung nach einem der vorhergehenden Ansprüche, die 1 bis 100 Gew.-% Fettphase enthält, welche Octyldodecylneopentanoat und die Siliconverbindungen und die Kohlenwasserstoffverbindungen enthält und 0 bis 99 Gew.-% einer Pulverphase enthält.

5. Zusammensetzung nach einem der vorhergehenden Ansprüche, in der die Siliconverbindungen ausgewählt sind unter Cyclomethiconen, wie Cyclomethicon D4, D5, D6, Polydimethylsiloxanen, Alkyldimethiconen, Polyphenylmethylsiloxanen, wie Phenyldimethiconen und Phenyltrimethiconen, Siliconen, die mit aliphatischen und/oder aromatischen, gegebenenfalls fluorierten Gruppen oder mit funktionellen Gruppen, wie Hydroxygruppen, Thiolgruppen und/oder Aminogruppen modifiziert sind; Siliconen der Formel (I) : 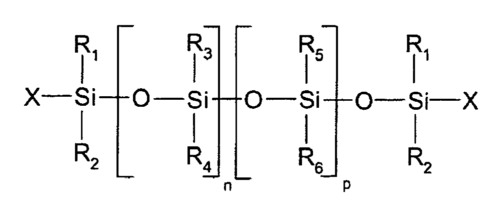 worin bedeuten:
R₁, R₂, R₅ und R₆, gleichzeitig oder unabhängig voneinander eine Alkylgruppe mit 1 bis 6 Kohlenstoffatomen,
R₃ und R₄, gleichzeitig oder unabhängig voneinander eine Alkylgruppe mit 1 bis 6 Kohlenstoffatomen oder eine Arylgruppe,
X eine Alkylgruppe mit 1 bis 6 Kohlenstoffatomen, eine Hydroxygruppe oder eine Vinylgruppe,
n und p Zahlen, die so ausgewählt sind, daß die Siliconverbindung eine Viskosität über 100 000 mPa·s aufweist.

6. Zusammensetzung nach einem der Ansprüche 1 bis 5, in der die Kohlenwasserstoffverbindung ausgewählt ist unter Bienenwachs, Carnaubawachs, Candellilawachs, Ouricuriwachs, Japanwachs, Wachsen von Korkfasern oder Zuckerrohrfasern, Paraffinwachsen, Lignit oder mikrokristallinen Wachsen oder Ozokeriten, Polyethylenwachsen oder Wachsen, die durch Fischer-Tropsch-Synthese hergestellt sind.

7. Zusammensetzung nach einem der vorhergehenden Ansprüche, wobei die Zusammensetzung Pigmente und/oder Füllstoffe enthält.

8. Zusammensetzung nach Anspruch 7, wobei die Pigmente unter den anorganischen, organischen Pigmenten und/oder Perlglanzpigmenten ausgewählt sind.

9. Zusammensetzung nach Anspruch 7, wobei die Füllstoffe unter den anorganischen, organischen oder synthetischen, lamellaren oder nichtlamellaren, sphärischen oder nichtsphärischen Füllstoffen ausgewählt sind.

10. Zusammensetzung nach einem der Ansprüche 7 bis 9, wobei die Pigmente und/oder Füllstoffe ausgewählt sind unter Titandioxid, Zinkoxid, Zirkoniumdioxid, Eisenoxiden, Cerdioxid, Chromoxid, Eisenblau, Ruß, Barium-, Strontium-, Calcium- und Aluminiumlacken, Glimmer, der mit Titanoxid oder Bismuthchloridoxid umhüllt ist, Talk, Glimmer, Siliciumoxid, Kaolin, Nylonpulvern und Polyethylenpulvern, Teflon, Stärke, Titandioxid, Glimmer, natürlichem Perlglanzpigment, Bornitrid, hohlen Mikrokügelschen und Mikrokügelchen aus Siliconharz.

11. Zusammensetzung nach einem der vorhergehenden Ansprüche, die als Pflegeprodukt und/oder Produkt zum Schminken der Haut und/oder der Keratinfasern vorliegt, wie als freies, gegossenes oder verpreßtes Pulver (Make-up, Wangenrouge, Lidschatten), als wasserfreies mehr oder weniger fluides Fettprodukt (Lippenstift, Mascara, Lack mit Lösungsmittel) als Öl oder Lotion für den Körper und/oder das Gesicht und als Produkt für das Haar, wie als wasserfreies Frisiergel.

12. Verwendung von Octyldodecylneopentanoat als Kompatibilisierungsmittel in einer kosmetischen wasserfreien Zusammensetzung, die mindestens eine Siliconverbindung, und eine Kohlenwasserstoffverbindung enthält, die von Octyldodecylneopentanoat verschieden ist und ausgewählt ist unter pflanzlichen, tierischen, mineralischen und/oder synthetischen Ölen oder Wachsen.
